# EUROPEAN PATENT APPLICATION

(11) **EP 4 545 224 A2**
(43) Date of publication of application: **30.04.2025**
(21) Application number: 24199521.6
(22) Date of filing: 10.09.2024
(51) Int. Cl.: B22F 12/90, B22F 10/28, B22F 10/85, B33Y 10/00, B33Y 30/00, B33Y 50/02, G01N 25/72, G01N 27/90, G01N 27/9013, G01N 27/904

(54) **IN-SITU MONITORING OF ADDITIVELY MANUFACTURED OBJECT USING AN EDDY CURRENT ARRAY PROBE**

(30) Priority: 23.10.2023 IN 202311072290
(71) Applicant: General Electric Company, Evendale, Ohio 45215 (US)
(72) Inventor: Kumar KM, Manoj, 560066 Bengaluru (IN); Sheila-Vadde, Aparna Chakrapani, 560066 Bengaluru (IN)
(74) Representative: Hafner & Kohl PartmbB

(57) **Abstract**

An additive manufacturing system (100) includes a recoater (126) and an eddy current array probe (ECAP) (154) coupled to the recoater (126). The ECAP (154) includes three rows (210, 212, 214) of sensor elements (302, 306, 310), and a drive line (320) arranged in a semi-circular wave pattern extending through each of the three rows (210, 212, 214) of sensor elements (302, 306, 310). The semi-circular wave pattern is defined by a wavelength (322), and the semi-circular wave pattern of the drive line (320) extending through a particular row (210, 212, 214) of sensor elements (302, 306, 310) is offset by a fraction of the wavelength (322) relative to the semi-circular wave pattern of the drive line (320) extending through an adjacent row (210, 212, 214) of sensor elements (302, 306, 310).

## Description

### PRIORITY INFORMATION

The present application claims priority to Indian Provisional Patent Application Serial Number 202311072290 filed on October 23, 2023.

### FIELD

The present disclosure generally pertains to additive manufacturing machines and systems, and more particularly, systems and methods for monitoring of an additively manufactured.

### BACKGROUND

Three-dimensional objects may be additively manufactured using an additive manufacturing machine. The additive manufacturing machine may perform a powder bed fusion process in which an energy beam system directs one or more energy beams onto a powder bed to consolidate (e.g., melt and/or sinter) sequential layers of powder material. The properties or qualities of the three-dimensional objects formed by consolidating the powder material may depend at least in part on the presence of internal imperfections or defects in the three-dimensional objects that occur during printing.

### BRIEF DESCRIPTION OF THE DRAWINGS

A full and enabling disclosure, including the best mode thereof, directed to one of ordinary skill in the art, is set forth in the specification, which makes reference to the appended figures, in which:
FIG. 1 schematically depicts an exemplary additive manufacturing system or machine in accordance with an embodiment of the present disclosure.
FIG. 2 schematically depicts a top plan view of a portion of the exemplary additive manufacturing system or machine of FIG. 1 in accordance with an embodiment of the present disclosure.
FIG. 3 schematically depicts an enlarged view of a portion of an eddy current array probe used in the exemplary additive manufacturing system or machine of FIGS. 1 and 2 in accordance with an embodiment of the present disclosure.
FIG. 4 is a block diagram of a computing system in accordance with various aspects of the present disclosure.
FIG. 5 is a block diagram depicting an embodiment of an in-situ method of monitoring and inspecting an additively manufactured object in accordance with various aspects of the present disclosure.

### DETAILED DESCRIPTION

Reference will now be made in detail to present embodiments of the disclosure, one or more examples of which are illustrated in the accompanying drawings. The detailed description uses numerical and letter designations to refer to features in the drawings. Like or similar designations in the drawings and description have been used to refer to like or similar parts of the disclosure.

The word "exemplary" is used herein to mean "serving as an example, instance, or illustration." Any implementation described herein as "exemplary" is not necessarily to be construed as preferred or advantageous over other implementations. Additionally, unless specifically identified otherwise, all embodiments described herein should be considered exemplary.

The singular forms "a," "an," and "the" include plural references unless the context clearly dictates otherwise.

The term "at least one of" in the context of, e.g., "at least one of A, B, or C" refers to only A, only B, only C, or any combination of A, B, and C.

As used herein, the terms "first," "second," and "third" may be used interchangeably to distinguish one component from another and are not intended to signify location or importance of the individual components.

The terms "coupled," "fixed," "attached to," and the like refer to both direct coupling, fixing, or attaching, as well as indirect coupling, fixing, or attaching through one or more intermediate components or features, unless otherwise specified herein.

The term "adjacent" as used herein with reference to two walls and/or surfaces refers to the two walls and/or surfaces contacting one another, or the two walls and/or surfaces being separated only by one or more nonstructural layers and the two walls and/or surfaces and the one or more nonstructural layers being in a serial contact relationship (i.e., a first wall/surface contacting the one or more nonstructural layers, and the one or more nonstructural layers contacting the a second wall/surface).

As used herein, the terms "additively manufactured" or "additive manufacturing techniques or processes" refer generally to manufacturing processes wherein successive layers of material(s) are provided on each other to "build-up," layer-by-layer, a three-dimensional component. The successive layers generally fuse together to form a monolithic component which may have a variety of integral sub-components. Although additive manufacturing technology is described herein as enabling fabrication of complex objects by building objects point-by-point, layer-by-layer, typically in a vertical direction, other methods of fabrication are possible and within the scope of the present subject matter. For example, although the discussion herein refers to the addition of material to form successive layers, one skilled in the art will appreciate that the methods and structures disclosed herein may be practiced with any additive manufacturing technique or manufacturing technology. For example, embodiments disclosed herein may use layer-additive processes, layer-subtractive processes, or hybrid processes.

Suitable additive manufacturing techniques in accordance with the present disclosure may include, but are not limited to, Fused Deposition Modeling (FDM), Selective Laser Sintering (SLS), 3D printing such as by inkjets, laser jets, and binder jets, Sterolithography (SLA), Direct Selective Laser Sintering (DSLS), Electron Beam Sintering (EBS), Electron Beam Melting (EBM), Laser Engineered Net Shaping (LENS), Laser Net Shape Manufacturing (LNSM), Direct Metal Deposition (DMD), Digital Light Processing (DLP), Direct Selective Laser Melting (DSLM), Selective Laser Melting (SLM), Direct Metal Laser Melting (DMLM), and other known processes.

The additive manufacturing processes described herein may be used for forming components using any suitable material. For example, the material may be plastic, metal, concrete, ceramic, polymer, epoxy, photopolymer resin, or any other suitable material that may be in solid, liquid, powder, sheet material, wire, or any other suitable form or combinations thereof. More specifically, according to exemplary embodiments of the present subject matter, the additively manufactured components described herein may be formed in part, in whole, or in some combination of materials including but not limited to pure metals, nickel alloys, chrome alloys, titanium, titanium alloys, magnesium, magnesium alloys, aluminum, aluminum alloys, and nickel or cobalt based superalloys or any electrically conducting materials (e.g., those available under the name Inconel^{®} available from Special Metals Corporation). These materials are examples of materials suitable for use in the additive manufacturing processes described herein, and may be generally referred to as "additive materials."

In addition, one skilled in the art will appreciate that a variety of materials and methods for bonding those materials may be used and are contemplated as within the scope of the present disclosure. As used herein, references to "fusing" may refer to any suitable process for creating a bonded layer of any of the above materials. For example, if an object is made from polymer, fusing may refer to creating a thermoset bond between polymer materials. If the object is epoxy, the bond may be formed by a crosslinking process. If the material is ceramic, the bond may be formed by a sintering process. If the material is powdered metal, the bond may be formed by a melting or sintering process. One skilled in the art will appreciate that other methods of fusing materials to make a component by additive manufacturing are possible, and the presently disclosed subject matter may be practiced with those methods.

In addition, the additive manufacturing process disclosed herein allows a single component to be formed from multiple materials. Thus, the components described herein may be formed from any suitable mixtures of the above materials. For example, a component may include multiple layers, segments, or parts that are formed using different materials, processes, and/or on different additive manufacturing machines. In this manner, components may be constructed which have different materials and material properties for meeting the demands of any particular application. In addition, although the components described herein are constructed entirely by additive manufacturing processes, it should be appreciated that in alternate embodiments, all or a portion of these components may be formed via casting, machining, and/or any other suitable manufacturing process. Indeed, any suitable combination of materials and manufacturing methods may be used to form these components.

An exemplary additive manufacturing process will now be described. Additive manufacturing processes fabricate components using three-dimensional (3D) information, for example a three-dimensional computer model, of the component. Accordingly, a three-dimensional design model of the component may be defined prior to manufacturing. In this regard, a model or prototype of the component may be scanned to determine the three-dimensional information of the component. As another example, a model of the component may be constructed using a suitable computer aided design (CAD) program to define the three-dimensional design model of the component.

The design model may include 3D numeric coordinates of the entire configuration of the component including both external and internal surfaces of the component. For example, the design model may define the body, the surface, and/or internal passageways such as openings, support structures, etc. In one exemplary embodiment, the three-dimensional design model is converted into a plurality of slices or segments, e.g., along a central (e.g., vertical) axis of the component or any other suitable axis. Each slice may define a thin cross section of the component for a predetermined height of the slice. The successive cross-sectional slices together form the 3D component. The component is then "built-up" slice-by-slice, or layer-by-layer, until finished.

In this manner, the components described herein may be fabricated using the additive process, or more specifically each layer is successively formed, e.g., by fusing or polymerizing a plastic using laser energy or heat or by sintering or melting metal powder. For example, a particular type of additive manufacturing process may use an energy beam, for example, an electron beam or electromagnetic radiation such as a laser beam, to sinter or melt a powder material. Any suitable laser and laser parameters may be used, including considerations with respect to power, laser beam spot size, and scanning velocity. The build material may be formed by any suitable powder or material selected for enhanced strength, durability, and useful life, particularly at high temperatures.

Each successive layer may be, for example, between about 10 micrometers (µm) and 200 µm, although the thickness may be selected based on any number of parameters and may be any suitable size according to alternative embodiments. Therefore, utilizing the additive formation methods described above, the components described herein may have cross sections as thin as one thickness of an associated powder layer, e.g., 10 µm, utilized during the additive formation process.

In addition, utilizing an additive process, the surface finish and features of the components may vary as need depending on the application. For example, the surface finish may be adjusted (e.g., made smoother or rougher) by selecting appropriate laser scan parameters (e.g., laser power, scan speed, laser focal spot size, etc.) during the additive process, especially in the periphery of a cross-sectional layer which corresponds to the part surface. For example, a rougher finish may be achieved by increasing laser scan speed or decreasing the size of the melt pool formed, and a smoother finish may be achieved by decreasing laser scan speed or increasing the size of the melt pool formed. The scanning pattern and/or laser power can also be changed to change the surface finish in a selected area.

Notably, in exemplary embodiments, several features of the components described herein were previously not possible due to manufacturing restraints. However, the present inventors have advantageously utilized current advances in additive manufacturing techniques to develop exemplary embodiments of such components generally in accordance with the present disclosure. While the present disclosure is not limited to the use of additive manufacturing to form these components generally, additive manufacturing does provide a variety of manufacturing advantages, including ease of manufacturing, reduced cost, greater accuracy, etc.

In this regard, utilizing additive manufacturing methods, even multi-part components may be formed as a single piece of continuous metal, and may thus include fewer sub-components and/or joints compared to prior designs. The integral formation of these multi-part components through additive manufacturing may advantageously improve the overall assembly process. For example, the integral formation reduces the number of separate parts that are assembled, thus reducing associated time and overall assembly costs. Additionally, existing issues with, for example, leakage, joint quality between separate parts, and overall performance may advantageously be reduced.

Also, the additive manufacturing methods described above enable much more complex and intricate shapes and contours of the components described herein. For example, such components may include thin additively manufactured layers and unique fluid passageways with integral mounting features. In addition, the additive manufacturing process enables the manufacture of a single component having different materials such that different portions of the component may exhibit different performance characteristics. The successive, additive nature of the manufacturing process enables the construction of these novel features. As a result, the components described herein may exhibit improved functionality and reliability.

Here and throughout the specification and claims, range limitations are combined and interchanged, such ranges are identified and include all the sub-ranges contained therein unless context or language indicates otherwise. For example, all ranges disclosed herein are inclusive of the endpoints, and the endpoints are independently combinable with each other.

Approximating language, as used herein throughout the specification and claims, is applied to modify any quantitative representation that could permissibly vary without resulting in a change in the basic function to which it is related. Accordingly, a value modified by a term or terms, such as "about", "approximately", and "substantially", are not to be limited to the precise value specified. In at least some instances, the approximating language may correspond to the precision of an instrument for measuring the value, or the precision of the methods or machines for constructing or manufacturing the components and/or systems. For example, the approximating language may refer to being within a 1, 2, 4, 10, 15, or 20 percent margin. These approximating margins may apply to a single value, either or both endpoints defining numerical ranges, and/or the margin for ranges between endpoints.

The present disclosure generally provides an apparatus and technique for in-situ monitoring and inspection of an additive manufacturing build using an eddy current array probe to detect or identify characteristics or anomalies of at least one or more consolidated layers of the additive build. In exemplary embodiments, the eddy current array probe may be configured as an omnidirectional eddy current array probe to detect anomalies in the additive manufacturing build disposed in varying directions. In exemplary embodiments, the eddy current array probe of the present disclosure is excitable at one or more frequencies to detect anomalies at varying depths. In exemplary embodiments, the eddy current array probe of the present disclosure can be embedded in or coupled to a recoater such that as powder material is spread for melting and fusing, inspection of the build layer(s) can be carried out. Additionally, embodiments of the present disclosure employ an algorithm using measurements from adjacent sensor elements to detect true anomalies and eliminate false readings.

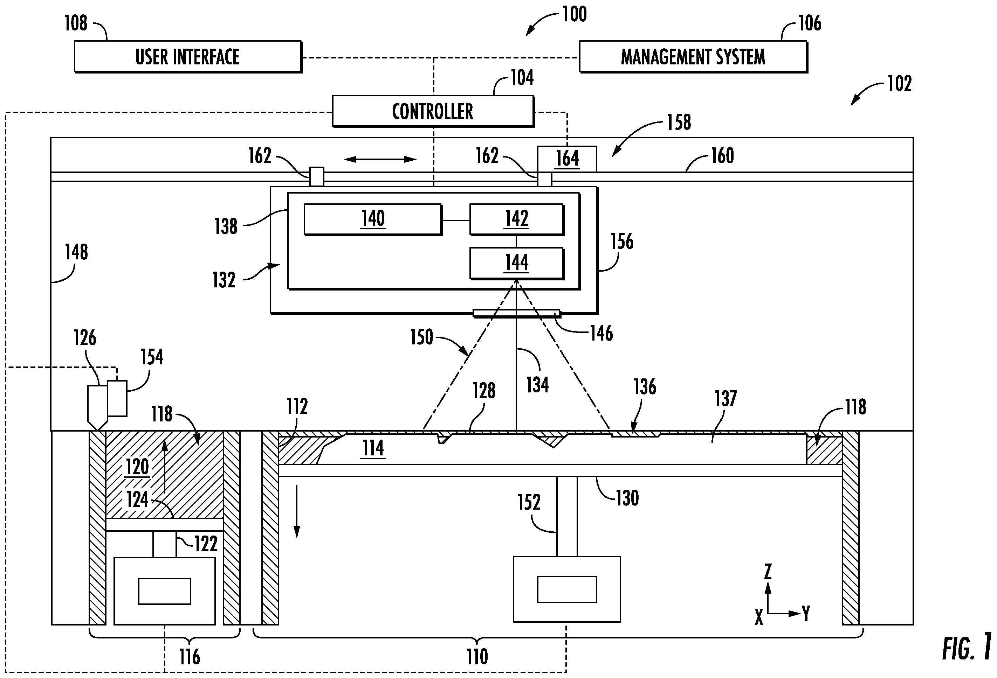

Referring now to FIG. 1, the presently disclosed subject matter will now be described in further detail. FIG. 1 schematically depicts an exemplary additive manufacturing system 100. The additive manufacturing system 100 may include one or more additive manufacturing machines 102. It will be appreciated that the additive manufacturing system 100 and additive manufacturing machine 102 shown in FIG. 1 are provided by way of example and not to be limiting. In fact, the subject matter of the present disclosure may be practiced with any suitable additive manufacturing system 100 and/or additive manufacturing machine 102 without departing from the scope of the present disclosure. As shown, the one or more additive manufacturing machines 102 may include a controller 104. The controller 104 may be associated with one or more additive manufacturing machines 102. The controller 104 may include componentry integrated as part of the additive manufacturing machine 102 and/or componentry that is provided separately from the additive manufacturing machine 102. Various componentry of the controller 104 may be communicatively coupled to various componentry of the additive manufacturing machine 102.

The controller 104 may be communicatively coupled with a management system 106 and/or a user interface 108. The management system 106 interact with the controller 104 in connection with enterprise-level operations pertaining to the additive manufacturing system 100. Such enterprise level operations may include transmitting data from the management system 106 to the controller 104 and/or transmitting data from the controller 104 to the management system 106. The user interface 108 may include one or more user input/output devices to allow a user to interact with the additive manufacturing system 100.

As shown, an additive manufacturing machine 102 may include a build module 110 that includes a build chamber 112 within which an object or objects 114 may be additively manufactured. An additive manufacturing machine 102 may include a powder module 116 that contains a supply of powder material 118 housed within a supply chamber 120. The build module 110 and/or the powder module 116 may be provided in the form of modular containers configured to be installed into and removed from the additive manufacturing machine 102 such as in an assembly-line process. Additionally, or in the alternative, the build module 110 and/or the powder module 116 may define a fixed componentry of the additive manufacturing machine 102.

The powder module 116 contains a supply of the powder material 118 housed within a supply chamber 120. The powder module 116 includes a powder piston 122 that elevates a powder floor 124 during operation of the additive manufacturing machine 102. As the powder floor 124 elevates, a portion of the powder material 118 is forced out of the powder module 116. A recoater 126, such as a blade or roller, sequentially distributes thin layers of the powder material 118 across a build plane 128 above the build module 110. A build platform 130 supports the sequential layers of the powder material 118 distributed across the build plane 128. A build platform 130 may include a build plate (not shown) secured thereto and upon which an object 114 may be additively manufactured.

The additive manufacturing machine 102 includes an energy beam system 132 configured to generate one or more of energy beams 134 and to direct the respective energy beams 134 onto the build plane 128 to selectively solidify respective portions of the powder bed 136 defining the build plane 128. The one or more energy beams 134 may be laser beams or beams from any other suitable energy source, such as LEDs or other light sources, and so forth. As a respective energy beam 134 selectively consolidates (e.g., melts or fuses) respective ones of the sequential layers of powder material 118 that define the powder bed 136, the object 114 begins to take shape (e.g., in the form of one or more consolidated layers 137 of the powder material 118). The one or more energy beams 134 may include electromagnetic radiation having any suitable wavelength or wavelength range, such as a wavelength or wavelength range corresponding to infrared light, visible light, and/or ultraviolet light.

Typically, with a DMLM, EBM, or SLM system, the powder material 118 is fully melted, with respective layers being melted or re-melted with respective passes of the energy beams 134. With DMLS or SLS systems, typically the layers of powder material 118 are sintered, fusing particles of powder material 118 to one another generally without reaching the melting point of the powder material 118. The energy beam system 132 may include componentry integrated as part of the additive manufacturing machine 102 and/or componentry that is provided separately from the additive manufacturing machine 102.

The energy beam system 132 may include one or more irradiation devices 138 respectively configured to generate one or more of energy beams 134 and to direct the one or more energy beams upon the build plane 128. The one or more irradiation devices may respectively include an energy beam source 140, an optical assembly 142, and a scanner 144. The optical assembly 142 may include a plurality of optical elements configured to direct an energy beam 134 onto the build plane 128. The optical assembly 142 may include one or more optical elements, such as lenses through which an energy beam 134 may be transmitted along an optical path from the energy beam source 140 to the build plane 128. By way of example, an optical assembly 142 may include one more focusing lenses that focus an energy beam 134 on the build plane 128. An exemplary scanner 144 may include a galvo scanner, an electro-optic modulator, an acousto-optic modulator, a piezo-driven mirror, or the like. In some embodiments, the energy beam system 132 may include a window 146, such as a protective glass, that separates one or more components of the energy beam system 132 from the environment of the process chamber 148. The window 146 may prevent contaminants from fumes associated with the additive manufacturing process, such as powder material, dust, soot, residues, vapor, splatter particles, byproducts, and the like, from coming into contact with sensitive components of an energy beam system 132.

The energy beam system 132 shown in FIG. 1 schematically depicts one irradiation device 138. In some embodiments, an energy beam system 132 may include a plurality of irradiation devices 138, such as two, three, four, six, eight, ten, or more irradiation devices 138, and such irradiation devices may respectively include an optical assembly 142. The plurality of irradiation devices 138 may respectively generate one or more energy beams 134 that are respectively scannable within a scan field 150 incident upon at least a portion of the build plane 128 to selectively consolidate the portions of the powder material 118 that thereby form part of the object 114.

To irradiate a layer of the powder bed 136, the one or more irradiation devices 138 respectively direct an energy beam 134 across the respective portions of the build plane 128 to selectively consolidate the portions of the powder material 118 that thereby forms part of the object 114. The one or more energy beams 134 may become incident upon the build plane 128 defined by the powder bed 136, for example, after passing through one or more optical elements of the optical assembly 142 and/or through the window 146 of the energy beam system 132. As sequential layers of the powder bed 136 are consolidated, a build piston 152 gradually lowers the build platform 130 to make room for sequential layers of powder material 118. As sequential layers of powder material 118 are applied across the build plane 128, the next sequential layer of powder material 118 defines the surface of the powder bed 136 coinciding with the build plane 128. Sequential layers of the powder bed 136 may be selectively consolidated until a completed object 114 has been additively manufactured. In some embodiments, an additive manufacturing machine 102 may utilize an overflow module (not shown) to capture excess powder material 118. Additionally, or in the alternative, excess powder material 118 may be redistributed across the build plane 128 when applying a next sequential layer of powder material 118. It will be appreciated that other systems may be provided for handling the powder material 118, including different powder supply systems and/or excess powder recapture systems. The subject matter of the present disclosure may be practiced with any suitable additive manufacturing machine 102 without departing from the scope hereof.

Still referring to FIG. 1, the additive manufacturing machine 102 includes an eddy current array probe (ECAP) 154 configured to detect anomalies in the build layers (e.g., the one or more consolidated layers 137). In the illustrated embodiment, the ECAP 154 is coupled to or configured as part of the recoater 126. However, it should be understood that the ECAP 154 may alternatively be configured as an independently movable device or be coupled to a different movable device (e.g., a movable powder delivery mechanism). Thus, in exemplary embodiments, the ECAP 154 is arranged on or in the recoater 126 such that the path of the ECAP 154 is identical with the path of movement of the recoater 126. A corresponding coupling of the movement of the ECAP 154 with the recoater 126 can be achieved by arranging or forming the ECAP 154 at least partially on or in the recoater 126. Arranging the ECAP 154 on or in the recoater 126 typically involves a material and/or nonpositive and/or positive structural connection between the ECAP 154 and the recoater 126, the connection possibly being reversible (without damage or without destruction). Formation of ECAP 154 on or in recoater 126 typically comprises structural integration of the ECAP 154 into the recoater 126. The ECAP 154 may also be adhesively coupled or bonded to the recoater 126.

The energy beam system 132 may be positioned at any suitable location within the process chamber 148. Additionally, or in the alternative, the energy beam system 132 may be coupled to a perimeter wall of the process chamber 148. In some embodiments, an additive manufacturing machine 102 may include a positioning system 158 configured to move an energy beam system 132 and/or one or more components thereof relative to the build plane 128. The positioning system 158 may move the energy beam system 132 and/or one or more components thereof to specified build coordinates and/or along specified build vectors corresponding to a coordinate system, such as a cartesian coordinate system, in accordance with control commands provided, for example, by the controller 104. The coordinate system may correspond to the build plane 128. Additionally, or in the alternative, the coordinate system may correspond to positions of the scanner 144. The control commands may be provided, for example, to carry out operations of the one or more energy beam system 132 and/or of the additive manufacturing machine 102 in accordance with the present disclosure. The positioning system 158 may include one or more gantry elements 160 configured to move the energy beam system 132 and/or one or more components thereof across the powder bed. Respective gantry elements 160 may move the energy beam system 132 and/or one or more components thereof in one or more directions, such as an X-direction, a Y-direction, and/or a Z-direction. In some embodiments, the positioning system 158 may be coupled to the housing assembly 156 that contains one or more components of the energy beam system 132. The housing assembly 156 may be coupled to one or more gantry elements 160 by one or more gantry mounts 162. The positioning system 158 may include a drive motor 164 configured to move the housing assembly 156 and/or the one or more components the energy beam system 132 according to instructions for the controller 104. The positioning system 158 may include componentry typically associated with a gantry system, such as stepper motors, drive elements, carriages, and so forth.

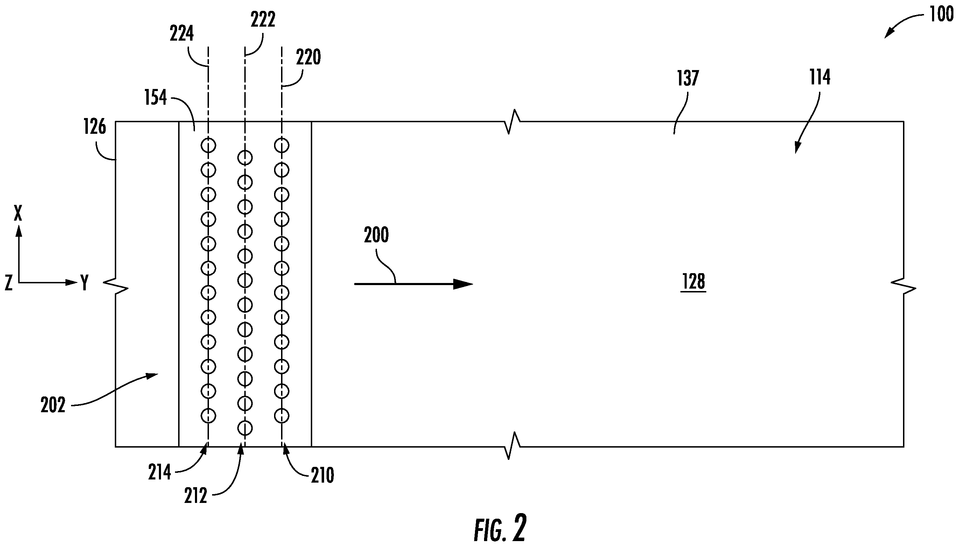

FIG. 2 is a schematic diagram depicting a top plan view of a portion of the additive manufacturing system 100 of FIG. 1 in accordance with an embodiment of the present disclosure. In the embodiment illustrated in FIG. 2, the recoater 126 and the ECAP 154 are arranged relative to the build plane 128 such that the recoater 126 and the ECAP 154 are movable across the build plane 128 in a scanning direction 200. During movement of the ECAP 154 in the scanning direction, the ECAP 154 is used to inspect one or more respective ones of the consolidated layers 137 forming the three-dimensional object 114. In the illustrated embodiment, the ECAP 154 provides two-dimensional coverage (e.g., extending in the X and Y axis directions) such that inspection of the one or more respective ones of the consolidated layers 137 is performed while the ECAP 154 is moved in the scanning direction 200.

Generally, eddy current inspection involves inducing eddy currents in the material to be tested and sensing the secondary magnetic fields generated by the eddy currents in the inspection material. By energizing a drive element above the surface of the inspection material, localized eddy currents are induced around the drive element footprint. The size, shape, and excitation frequency of the drive element affects the eddy current density profile generated on the material to be inspected. A passive sense element adjacent to the energized drive element can detect the variation in a secondary magnetic field whenever a defect distorts the secondary magnetic field. According to embodiments of the present disclosure, as set forth further below, by having large number of passive sense element arrays and routing the drive element around the sense element array enables the inspection of a large area in very short duration.

In the illustrated embodiment, the ECAP 154 includes a sensor array 202 for inducing localized eddy currents into the consolidated layers 137 and detecting response signals from the induced eddy currents. In the illustrated embodiment, the sensor array 202 includes a first row sensor element group 210, second row sensor element group 212, and a third row sensor element group 214. The first row sensor element group 210 is arranged along a first linear axis 220, the second row sensor element group 212 is arranged along a second linear axis 222, and the third row sensor element group 214 is arranged along a third linear axis 224. In exemplary embodiments, each of the first linear axis 220, the second linear axis 222, and the third linear axis 224 are disposed or oriented orthogonal or perpendicular to the scanning direction 200. Additionally, in exemplary embodiments, the first linear axis 220, the second linear axis 222, and the third linear axis 224 are each offset from each other in a lateral direction corresponding to the scanning direction 200.

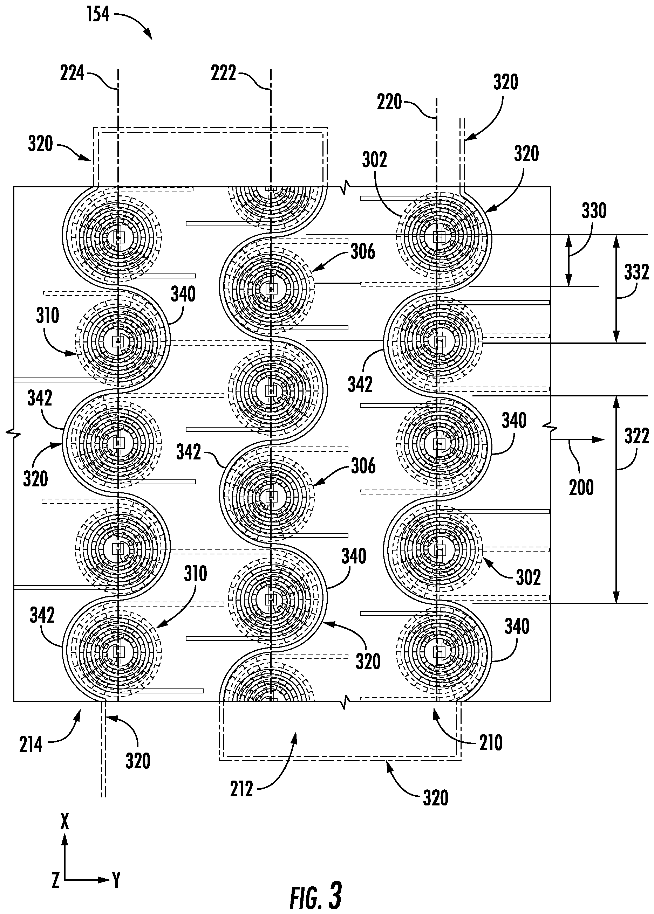

FIG. 3 is an enlarged schematic view of a portion of the ECAP 154 of FIGS. 1 and 2 in accordance with an embodiment of the present disclosure. In the embodiment illustrated in FIG. 3, the first row sensor element group 210 includes one or more first sensor elements 302 each arranged along the first linear axis 220. The second row sensor element group 212 includes one or more second sensor elements 306 each arranged along the second linear axis 222. The third row sensor element group 214 includes one or more third sensor elements 310 each arranged along the third linear axis 224. In the illustrated embodiment, the respective one or more first, second, and third sensor elements 302, 306, and 310 are spiral shaped sensor elements 302, 306, and 310. However, the particular shape and configuration of the sensor elements 302, 306, and 310 is not intended to be limited. It should also be understood that sensing lines or wires of the sensor elements 302, 306, and 310 may be composed of multiple turns in the same layer or in multiple layers as disclosed and described in U.S. Patent No. 7,948,233 and incorporated herein by reference in its entirety.

In exemplary embodiments, the ECAP 154 also includes a continuous drive line 320 extending along each of the first row sensor element group 210, the second row sensor element group 212, and the third row sensor element group 214. The drive line 320 is configured as or provides a semi-circular wave pattern (e.g., forming a sinusoidal-like waveform) operable to induce an eddy current in the consolidated layers 137. In the illustrated embodiment, the drive line 320 generally comprises a wavelength 322, and the drive line 320 is offset in the respective first row sensor element group 210, the second row sensor element group 212, and the third row sensor element group 214. For example, the semi-circular wave pattern of the drive line 320 extending through a particular row of sensor elements is offset by a fraction of the wavelength 322 relative to the semi-circular wave pattern of the drive line 320 extending through an adjacent row of sensor elements. In the illustrated embodiment, the semi-circular wave pattern of the drive line 320 extending along the second row sensor element group 212 is offset from the semi-circular wave pattern of the drive line 320 extending along the first row sensor element group 210 by one quarter of the wavelength 322 (i.e., ninety (90) degrees), indicated by a dimension 330 in FIG. 3. Further, the semi-circular wave pattern of the drive line 320 extending along the third row sensor element group 214 is offset from the semi-circular wave pattern of the drive line 320 extending along the first row sensor element group 210 by one half of the wavelength 322 (i.e., one hundred eighty (180) degrees), indicated by a dimension 332 in FIG. 3. The semi-circular wave pattern of the drive line 320 extends laterally generally in the direction of the first linear axis 220, the second linear axis 222, and the third linear axis 224, respectively, such that one or more peaks 340 of the drive line 320 and one or more troughs 342 of the drive line 320 extend laterally in the scanning direction 200. As described above, the drive line 320 is a continuous drive line 320 such that the drive line 320 extends from the first row sensor element group 210 to the second row sensor element group 212, and from the second row sensor element group 212 to the third row sensor element group 214.

In the illustrated embodiment, the first, second, and third sensor elements 302, 306, and 310 are at least partially disposed within a parameter defined by the drive line 320 and are offset from each other in such a way to avoid blind zones for defect or anomaly detection in any orientation. For example, the first, second, and third sensor elements 302, 306, and 310 are at least partially disposed within a parameter defined by the drive line 320 and are offset by ninety (90) degrees of the wavelength 322 from a sensor element of an adjacent sensor element group row. For example, the first sensor elements 302 are offset by ninety (90) degrees of the wavelength 322 from the second sensor elements 306. Similarly, the third sensor elements 310 are offset by ninety (90) degrees of the wavelength 322 from the second sensor elements 306. As depicted in the illustrated embodiment, the semi-circular wave pattern of the drive line 320 directionally alternates to opposite sides of adjacently disposed sensor elements 302, 306, and 310, thereby forming a sinusoidal-like waveform along the respective sensor elements 302, 306, and 310.

Generally, crack orientation with respect to a drive element/eddy current direction can influence detection sensitivity. For example, maximum field disturbance is achieved when the crack length is perpendicular to the eddy current flow. However, when the crack length is parallel to the eddy current flow, there is minimal disturbance to the current flow, and it becomes difficult to detect the crack. In accordance with exemplary embodiments of the present disclosure, to detect cracks in different directions, the ECAP 154 is configured as an omnidirectional ECAP 154 to ensure sensitivity to cracks in all orientations and avoids any blind zones.

Thus, as described above, in the embodiment illustrated in FIG. 3, each sensor element 302, 306, and 310 is spatially offset laterally in a direction orthogonal or perpendicular to the scanning direction 200 from a respective sensor element 302, 306, and 310 in an adjacent row by a distance equal to a quarter of the wavelength 322 formed by the drive line 320, thereby providing omnidirectional flaw detection. The spatial offset between sensor elements in adjacent rows ensures a minimum response for a crack orientation from one sensor element gets automatically compensated with a maximum from the sensor element in the adjacent sensor element row. In other words, the offset configuration of the drive line 320 and the sensor elements 302, 306, and 310 in each sensor element group row (e.g., the first row sensor element group 210, the second row sensor element group 212, and the third row sensor element group 214) ensures that there are no gaps between sensor elements and that a flaw or anomaly is detected by one or more of the sensor elements 302, 306, and 310 in one or more rows of sensor element groups. In the illustrated embodiment, three rows of sensor element groups are illustrated to provide omnidirectional flaw detection (e.g., 0°, +45°, -45°, and 90° crack orientation relative to the build plane 128 (FIGS. 1 and 2)). However, if only parallel and perpendicular flaws are of interest relative to the build plane 128 (FIGS. 1 and 2), then only two rows of sensor element groups are needed.

In operation, as the ECAP 154 is moved in the scanning direction 200 across the build plane 128 (FIGS. 1 and 2), the drive line 320 is excited at one or more frequencies. For example, lower excitation frequencies are useful for detecting deeper defects while higher excitation frequencies are useful in detecting surface or near-surface defects. Thus, in operation, the ECAP 154 in combination with multifrequency excitation detects anomalies buried within multiple layers of the build (e.g., at least three to four layers of the build). In exemplary embodiments, the drive line 320 is excited at one or more frequencies ranging from about 100 kHz to about 6 MHz. The multifrequency excitation provides signatures of anomalies buried within at least about 500 µm depth from a surface of the inspection.

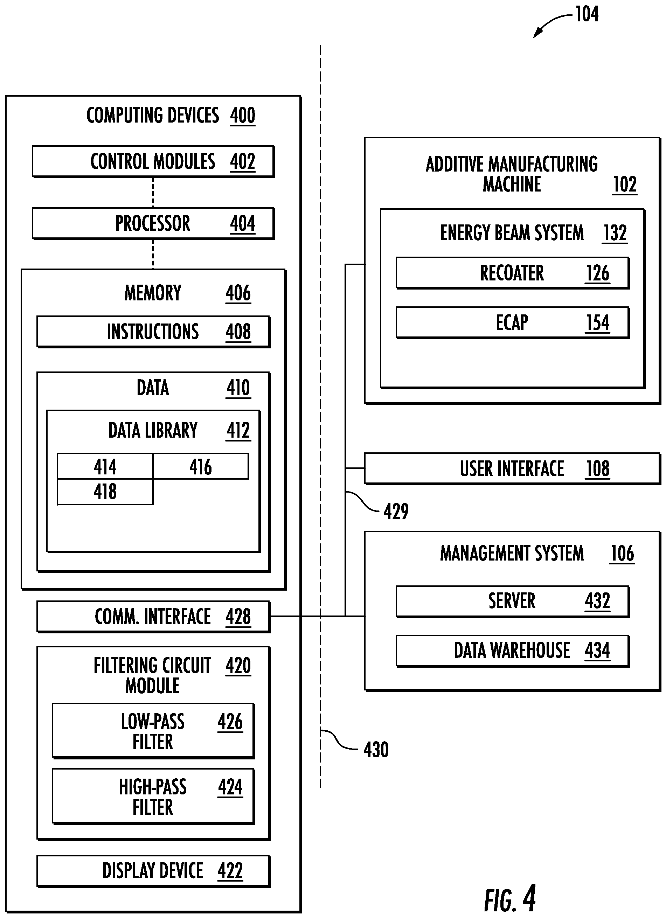

FIG. 4 depicts an exemplary controller 104 in accordance with exemplary embodiments of the present disclosure. The controller 104 may include one or more computing devices 400. The one or more computing devices 400 may be communicatively coupled with the additive manufacturing machine 102 (FIG. 1) and additive manufacturing system 100 (FIG. 1). For example, the computing device 400 may be communicatively coupled with one or more control modules 402 for controlling one or more controllable components associated with the additive manufacturing machine 102 (FIG. 1) and additive manufacturing system 100 (FIG. 1), such as, but not limited to, the energy beam system 132 (FIG. 1), the recoater 126 (FIGS. 1 and 2), and the ECAP 154 (FIGS. 1-3). Additionally, or in the alternative, the computing device 400 may be communicatively coupled with the management system 106 (FIG. 1) and the user interface 108 (FIG. 1). The one or more computing devices 400 may be located locally or remotely relative to the additive manufacturing machine 102 (FIG. 1).

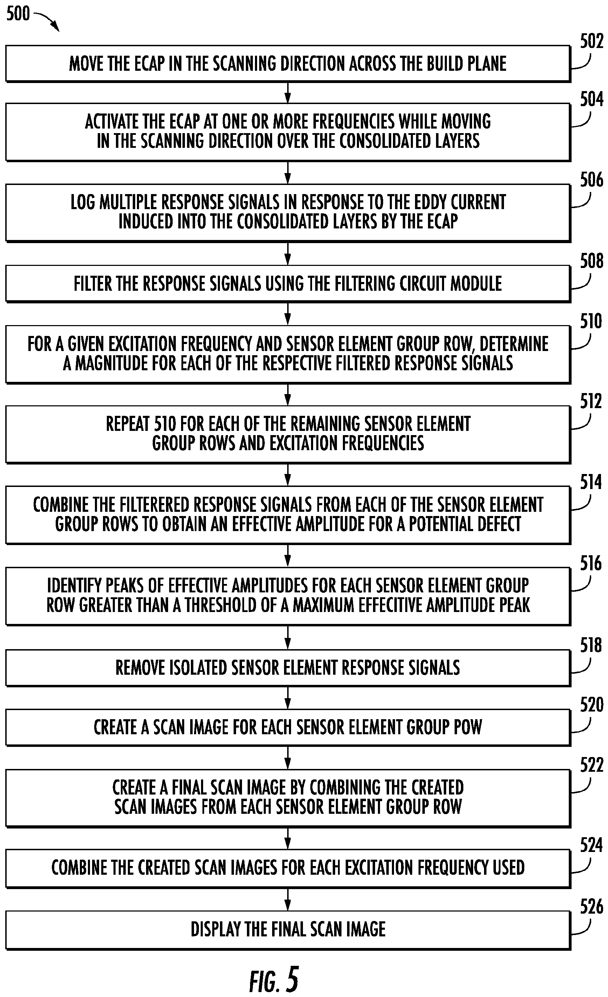

The one or more computing devices 400 may include the one or more control modules 402 configured to cause the computing device 400 to perform one or more control operations. The one or more control modules 402 may include control logic executable to provide control commands configured to perform control operations associated with controlling one or more controllable components of the additive manufacturing machine 102 (FIG. 1). Additionally, or in the alternative, the one or more control modules 402 may perform one or more methods described with reference to FIG. 5.

The one or more computing devices 400 may include one or more processors 404 and one or more memory devices 406. The one or more processors 404 may include any suitable processing device, such as a microprocessor, microcontroller, integrated circuit, logic device, and/or other suitable processing device. The one or more memory devices 406 may include one or more computer-readable media, including but not limited to non-transitory computer-readable media, RAM, ROM, hard drives, flash drives, and/or other memory devices 406. The one or more control modules 402 may be implemented at least in part by the one or more processors 404 or the one or more memory devices 406.

As used herein, the terms "processor" and "computer" and related terms, such as "processing device" and "computing device", are not limited to just those integrated circuits referred to in the art as a computer, but broadly refers to a microcontroller, a microcomputer, a programmable logic controller (PLC), an application specific integrated circuit, and other programmable circuits, and these terms are used interchangeably herein. The memory device 406 may include, but is not limited to, a non-transitory computer-readable medium, such as a random-access memory (RAM), and computer-readable nonvolatile media, such as hard drives, flash memory, and other memory devices. Alternatively, a floppy disk, a compact disc-read only memory (CD-ROM), a magneto-optical disk (MOD), and/or a digital versatile disc (DVD) may also be used.

As used herein, the term "non-transitory computer-readable medium" is intended to be representative of any tangible computer-based device implemented in any method or technology for short-term and long-term storage of information, such as, computer-readable instructions, data structures, program modules and sub-modules, or other data in any device. The methods described herein may be encoded as executable instructions embodied in a tangible, non-transitory, computer readable media, including, without limitation, a storage device and/or a memory device. Such instructions, when executed by a processor, cause the processor to perform at least a portion of the methods described herein. Moreover, as used herein, the term "non-transitory computer-readable medium" includes all tangible, computer-readable media, including, without limitation, non-transitory computer storage devices, including, without limitation, volatile and nonvolatile media, and removable and non-removable media such as a firmware, physical and virtual storage, CD-ROMs, DVDs, and any other digital source such as a network or the Internet, as well as yet to be developed digital means, with the sole exception being a transitory, propagating signal.

The one or more memory devices 406 may store information accessible by the one or more processors 404, including computer-executable or computer-readable instructions 408 that can be executed by the one or more processors 404. The instructions 408 may include any set of instructions which when executed by the one or more processors 404 cause the one or more processors 404 to perform operations, including scanning and eddy-current excitation and anomaly detection operations, or additive manufacturing operations. For example, the instructions 408 may cause the one or more processors 404 to perform operations associated with the ECAP 154 such as described with reference to FIGS. 1-3. Additionally, or in the alternative, the instructions 408 may cause the one or more processors 404 to perform one or more methods, such as described with reference to FIG. 5.

The memory devices 406 may store data 410 accessible by the one or more processors 404. The data 410 may be stored in a data library 412. As examples, the data 410 may include one or more frequencies 414 used in exciting or activating the ECAP 154 (FIGS. 1-3), response signals 416 received by the ECAP 154 in response to induced eddy currents into the inspection material (e.g., the consolidated layers 137 (FIG. 1)) by the ECAP 154 (FIGS. 1-3), and image data 418 representing or depicting anomalies in the inspected material based on the response signals 416. By way of example, the data 410 may also include inputs to the ECAP 154 (FIGS. 1-3) or the recoater 126 (FIGS. 1 and 2) such as scanning speed, frequency signals, and other data sets, parameters, outputs, information, associated with the additive manufacturing system 100 (FIG. 1) or the additive manufacturing machine 102 (FIG. 1).

The one or more computing devices 400 may also include a filtering circuit module 420 and a display device 422. The filtering circuit module 420 comprises a signal processing device or circuit for processing or filtering the response signals 416. For example, in exemplary embodiments, the filtering circuit module 420 may comprise a high pass filter 424 and a low pass filter 426. The high pass filter 424 allows high signal values to pass and filters out the low signal values, and the low pass filter 426 allows low signal values to pass and filters out the high signal values. Thus, in operation, the response signals 416 are processed using the high pass filter 424 and the low pass filter 426 to remove noise. The display device 422 may comprise any suitable device for displaying a graphical representation of any anomalies detected using the ECAP 154 (FIGS. 1-3). The display device 422 may, additionally or alternatively, be a part of the user interface 108.

The one or more computing devices 400 may also include a communication interface 428 configured to communicate with various nodes on a communication network 430 via wired or wireless communication lines 429. The communication interface 428 may include any suitable components for interfacing with one or more network(s), including for example, transmitters, receivers, ports, controllers, antennas, and/or other suitable components. The communication interface 428 may allow the one or more computing devices 400 to communicate with various nodes associated with the additive manufacturing machine 102, the management system 106, and the user interface 108. The communication network 430 may include, for example, a local area network (LAN), a wide area network (WAN), SATCOM network, VHF network, a HF network, a Wi-Fi network, a WiMAX network, a gatelink network, and/or any other suitable communication network 430 for transmitting messages to and/or from the computing device 400 across the communication lines. The communication lines of the communication network 430 may include a data bus or a combination of wired and/or wireless communication links.

The management system 106 may include a server 432 and/or a data warehouse 434. As an example, at least a portion of the data 410 may be stored in the data warehouse 434, and the server 432 may transmit the data 410 from the data warehouse 434 to the one or more computing device 400, and receive the data 410 from the one or more computing devices 400 and to store the received data 410 in the data warehouse 434 for further purposes. The server 432 and/or the data warehouse 434 may be implemented as part of the one or more computing devices 400 or as part of the management system 106.

Thus, referring to FIGS. 1-4, in exemplary embodiment operations, as the recoater 126 spreads powder material 118 across or over the build plane 128 for melting and fusing, the ECAP 154 moves concurrently with the recoater 126 over the build plane 128. The ECAP 154 may be moved over the build plane 128 at a variety of different speeds (e.g., from about 0.1 mm/s to about 50 mm/sec). While the ECAP 154 is moving across the build plane 128 in the scanning direction 200, the ECAP 154 is activated to inspect the fused layer(s) (e.g., the consolidated layer(s) 137) beneath the powder bed 136. In exemplary embodiments, as the ECAP 154 is moved across the build plane 128, the drive line 320 is excited at one or more frequencies. Each of the first sensor elements 302, second sensor elements 306, and third sensor elements 310 logs multiple response signals 416 in the form of real and imaginary voltage amplitude values every second in response to the eddy currents induced into the consolidated layer(s) 137 by the ECAP 154. As described above, three rows of sensor element groups with a continuous drive line 320 extending along the three rows of sensor element groups as defined herein enables detection of defects independent of their orientation with respect to the scanning direction 200. The response signals 416 corresponding to each of the first row sensor element group 210, the second row sensor element group 212, and the third row sensor element group 214 are then filtered using the filtering circuit module 420 (i.e., filtering the response signals 416 using the high pass filter 424 and the low pass filter 426). For a given excitation frequency 414, real and imaginary components of the filtered response signals 416 from the first row sensor element group 210 can be combined together through vector addition to provide a magnitude for each of the respective response signals 416. The real and imaginary components together also provide the phase of the respective response signals 416. This vector addition procedure is repeated for each of the second row sensor element group 212 and the third row sensor element group 214. Response signals 416 from each of the three sensor element group rows can then be combined to obtain an effective amplitude for a potential defect based on the magnitudes determined for the respective response signals 416.

In exemplary embodiments, peaks of the effective amplitudes of the filtered response signals 416 from each of the first row sensor element group 210, the second row sensor element group 212, and the third row sensor element group 214 are detected and identified corresponding to a detection threshold (e.g., amplitudes greater than 25% of the peak effective amplitude). For example, response signals 416 from a defect are typically picked up by more than one sensor element in one or more sensor element group rows (e.g., neighboring or adjacent sensor elements in the same row, neighboring sensor elements of an adjacent row, or both). In such cases, after thresholding, there may be some spurious signals. Usually, these signals are seen in only one sensor element response (e.g., isolated sensor element response signals 416) and those response signals 416 can be removed, thereby helping reduce false positives. Thus, in exemplary embodiments, the evaluation of the response signals 416 may include determining whether response signals 416 indicative of an anomaly that are based on response signals 416 corresponding to a particular sensor element or sensor element group row are also reflected in response signals 416 corresponding to another sensor element or sensor element group row. In some embodiments the phase of the response signals 416 may be used in the discrimination of false positives which can have a different phase angle compared to phase angle of a defect. The process described above is repeated for each excitation frequency used with the ECAP 154.

The processed response signals 416 from the first row sensor element group 210 may be used to create a first scan image, the processed response signals 416 from the second row sensor element group 212 may be used to create a second scan image, and the processed response signals 416 from the third row sensor element group 214 may be used to create a third scan image. For creating a final scan image, the first, second, and third scan images are combined. Further, final scan images from multiple frequencies can be combined using different methods to get a resultant final scan image where real defects or anomalies are highlighted, and false indications are reduced or removed.

FIG. 5 provides a flow diagram of an exemplary method (500) for in-situ monitoring of an additive build in accordance with exemplary embodiments of the present disclosure. It should be appreciated that the method (500) is discussed herein only to describe exemplary aspects of the present subject matter and is not intended to be limiting.

At (502), the method (500) includes moving the ECAP 154 in the scanning direction 200 across the build plane 128. At (504), the method (500) includes activating the ECAP 154 at one or more frequencies while moving in the scanning direction 200 over the consolidated layers 137. At (506), the method (500) includes logging multiple response signals 416 in the form of real and imaginary voltage amplitude values in response to the eddy currents induced into the consolidated layer(s) 137 by the ECAP 154. At (508), the method (500) includes filtering the response signals 416 using the filtering circuit module 420.

At (510), the method (500) includes, for a given excitation frequency 414 and sensor element group row (e.g., the first row sensor element group 210), combining real and imaginary components through vector addition to determine a magnitude for each of the respective response signals 416. At (512), method (500) at (510) is repeated for each of the remaining sensor element group rows and excitation frequencies. At (514), the method (500) includes combining the respective response signals 416 from each of the three sensor element group rows to obtain an effective amplitude for a potential defect based on the magnitudes determined at (510). At (516), method (500) includes identifying peaks of the effective amplitudes of the filtered response signals 416 greater than a detection threshold of a maximum effective amplitude peak. At (518), method (500) includes removing isolated sensor element response signals 416. At (520), method (500) includes creating a scan image from the processed response signals 416 for each sensor element group row. At (522), method (500) includes creating a final scan image by combining the scan images created at (520). At (524), method (500) includes combining the created scan images for each excitation frequency used. At (526), method (500) includes displaying the final scan image.

It should be understood that the above-referenced method (500) for in-situ monitoring of an additive build in accordance with exemplary embodiments of the present disclosure may be performed using a variety of configurations of eddy current array probes. For example, the above-referenced method (500) may be used to process response signals induced by eddy currents using the eddy current array probe disclosed and described in U.S. Patent No. 7,948,233 (incorporated herein by reference in its entirety).

Thus, building layer by layer using additive manufacturing techniques is prone to defect formation within the formed components. The defect types include unfused powders, delamination, cracks, voids and such. Detecting these defects during the build can potentially improve throughput and reduce powder wastage. Additionally, inspection and qualification of large parts is challenging and often suffers from lack of a satisfactory monitoring and inspection technique. Inspecting and qualifying each layer in-situ and thereby the finished component provides definite advantages. Embodiments of the present disclosure provide an in-situ technique that can support real time build control, which provides considerable value. Embodiments of the present disclosure provide an eddy current array probe sensor for in-situ monitoring of an additive build. Exemplary embodiments of the present disclosure provide inspection and monitoring of build quality of multiple layers. In exemplary embodiments, the eddy current array probe can be embedded in or coupled to the recoater such that as the powder is spread for melting and fusing, inspection of the build layer can be carried out.

Thus, embodiments of the present disclosure provide an in-situ method of monitoring and inspecting an additive build using an eddy current array probe. The eddy current array probe can be configured as an omnidirectional eddy current array probe to detect anomalies in the build disposed in varying directions. In exemplary embodiments, the eddy current array probe of the present disclosure is excitable at different frequencies to detect anomalies at varying depths. Additionally, embodiments of the present disclosure employ an algorithm using measurements from adjacent sensor elements to detect true anomalies and eliminate false readings.

Further aspects of the presently disclosed subject matter are provided by the following clauses:
A system for monitoring an additive manufacturing build, the system comprising: a recoater; and an eddy current array probe (ECAP) coupled to the recoater, wherein the ECAP comprises: a first row sensor element group; a second row sensor element group; a third row sensor element group; and a drive line arranged in a semi-circular wave pattern extending through each of the first, second, and third sensor element groups, wherein the semi-circular wave pattern is defined by a wavelength, and wherein the drive line extending through the second row sensor element group is offset by one quarter of the wavelength from the drive line extending through the first row sensor element group, and wherein the drive line extending through the third row sensor element group is offset by one half of the wavelength from the drive line extending through the first row sensor element group.

The system of the preceding clause, further comprising a controller configured to: move the recoater in s scanning direction; during at least a portion of the recoater being moved in the scanning direction, energize the drive line to generate a plurality of response signals based on eddy currents induced into the one or more consolidated layers by the drive line; and process the plurality of response signals to obtain a representation of at least one characteristic of the one or more consolidated layers.

The system of any preceding clause, wherein the controller is configured to process the plurality of response signals by passing each of the plurality of response signals through at least one of a high-pass filter or a low-pass filter.

The system of any preceding clause, wherein the controller is configured to energize the drive line at one or more frequencies

The system of any preceding clause, wherein the controller is configured to: energize the drive line at one or more frequencies; and for each frequency of the one or more frequencies, obtain data from at least the first row sensor element group and combine the obtained data.

The system of any preceding clause, wherein: the first row sensor element group comprises one or more first sensor elements arranged along a first linear axis;
the second row sensor element group comprises on or more second sensor elements arranged along a second linear axis; and the third row sensor element group comprises one or more third sensor elements arranged along a third linear axis, wherein the first, second, and third linear axes are orthogonal to a scanning direction.

The system of any preceding clause, wherein the first, second, and third linear axes are offset from each other in the scanning direction.

A method of monitoring an additive manufacturing build, the method comprising: moving a recoater in a scanning direction over at least a portion of one or more consolidated layers of a powder material on a build platform, wherein the recoater comprises an eddy current array probe (ECAP); during at least a portion of the moving of the recoater, energizing the ECAP to generate a plurality of response signals based on eddy currents induced into the one or more consolidated layers by the ECAP; determining a magnitude of each of the plurality of response signals; determining an effective amplitude of a potential anomaly in the one or more consolidated layers based on the determined magnitude of each of the plurality of response signals; and verifying that the effective amplitude exceeds a threshold.

The method of any preceding clause, further comprising energizing the ECAP at one or more frequencies.

The method of any preceding clause, further comprising evaluating a phase angle of the plurality of response signals to determine the potential anomaly.

The method of any preceding clause, further comprising passing each of the plurality of response signals through at least one of a high-pass filter or a low-pass filter.

The method of any preceding clause, wherein the ECAP comprises a plurality of sensor element group rows, and wherein the method further comprises determining whether the potential anomaly is detected in at least two of the plurality of sensor element group rows.

The method of any preceding clause, further comprising: energizing the ECAP at one or more frequencies; and determining, for each of the one or more frequencies, the magnitude of each of the plurality of response signals.

The method of any preceding clause, wherein the ECAP comprises a plurality of sensor element group rows, and wherein the method further comprises: creating a scan image for each of the plurality of sensor element group rows; and combining the scan image from each of the plurality of sensor element group rows to form a final scan image.

A non-transitory computer-readable medium comprising computer-executable instructions, which, when executed by a processor associated with an additive manufacturing machine, causes the processor to perform a method comprising: moving a recoater in a scanning direction to spread layers of powder over a build platform, the recoater comprising an eddy current array probe (ECAP); irradiating the layers of powder to form one or more consolidated layers; during at least a portion of the moving of the recoater, energizing the ECAP to generate a plurality of response signals based on eddy currents induced into the one or more consolidated layers by the ECAP; determining a magnitude of each of the plurality of response signals; determining an effective amplitude of a potential anomaly in the one or more consolidated layers based on the determined magnitude of each of the plurality of response signals; and verifying that the effective amplitude exceeds a threshold.

The computer-readable medium of any preceding clause, wherein the computer-executable instructions, which when executed by the processor, causes the processor to perform the method comprising energizing the ECAP at one or more frequencies.

The computer-readable medium of any preceding clause, wherein the computer-executable instructions, which when executed by the processor, causes the processor to perform the method comprising passing each of the plurality of response signals through at least one of a high-pass filter or a low-pass filter.

The computer-readable medium of any preceding clause, wherein the computer-executable instructions, which when executed by the processor, causes the processor to perform the method comprising: energizing the ECAP at one or more frequencies; and determining, for each of the one or more frequencies, the magnitude of each of the plurality of response signals.

The computer-readable medium of any preceding clause, wherein the ECAP comprises a plurality of sensor element group rows, and wherein the computer-executable instructions, and when executed by the processor, causes the processor to perform the method comprising determining whether the potential anomaly is detected in at least two of the plurality of sensor element group rows.

The computer-readable medium of any preceding clause, wherein the computer-executable instructions, which when executed by the processor, causes the processor to perform the method comprising: energizing the ECAP at one or more frequencies; and determining, for each of the one or more frequencies, the magnitude of each of the plurality of response signals.

The computer-readable medium of any preceding clause, wherein the ECAP comprises a plurality of sensor element group rows, and wherein the computer-executable instructions, which when executed by the processor, causes the processor to perform the method comprising: creating a scan image for each of the plurality of sensor element group rows; and combining the scan image from each of the plurality of sensor element group rows to form a final scan image.

An eddy current array probe, comprising: a first row sensor element group; a second row sensor element group; a third row sensor element group; and a drive line arranged in a semi-circular wave pattern extending through each of the first, second, and third sensor element groups, wherein the semi-circular wave pattern is defined by a wavelength, and wherein the drive line extending through the second row sensor element group is offset by one quarter of the wavelength from the drive line extending through the first row sensor element group, and wherein the drive line extending through the third row sensor element group is offset by one half of the wavelength from the drive line extending through the first row sensor element group.

The eddy current array probe of any preceding clause, wherein: the first row sensor element group comprises one or more first sensor elements arranged along a first linear axis; the second row sensor element group comprises on or more second sensor elements arranged along a second linear axis; and the third row sensor element group comprises one or more third sensor elements arranged along a third linear axis;
The eddy current array probe of any preceding clause, wherein the first, second, and third linear axes are orthogonal to the scanning direction.

The eddy current array probe of any preceding clause, wherein the first, second, and third linear axes are offset from each other in the scanning direction.

The eddy current array probe of any preceding clause, wherein the drive line extends from the first row sensor element group to the second row sensor element group, and wherein the drive line extends from the second row sensor element group to the third row sensor element group.

This written description uses examples to describe the presently disclosed subject matter, including the best mode, and also to enable any person skilled in the art to practice such subject matter, including making and using any devices or systems and performing any incorporated methods. The patentable scope of the presently disclosed subject matter is defined by the claims, and may include other examples that occur to those skilled in the art. The scope of the claims encompasses such other examples that include structural elements that do not differ from the literal language of the claims or that have insubstantial differences from the literal languages of the claims.

## Claims

1. An additive manufacturing system (100), comprising:
a recoater (126); and
an eddy current array probe (ECAP) (154) coupled to the recoater (126), wherein the ECAP (154) comprises:
a first row sensor element group (210);
a second row sensor element group (212);
a third row sensor element group (214); and
a drive line (320) arranged in a semi-circular wave pattern extending through each of the first, second, and third sensor element groups (210, 212, 214), wherein the semi-circular wave pattern is defined by a wavelength (322), and wherein the drive line (320) extending through the second row sensor element group (212) is offset by one quarter of the wavelength (322) from the drive line (320) extending through the first row sensor element group (210), and wherein the drive line (320) extending through the third row sensor element group (214) is offset by one half of the wavelength (322) from the drive line (320) extending through the first row sensor element group (210).

2. The system (100) of claim 1, further comprising a controller (104) configured to:
move the recoater (126) in a scanning direction;
during at least a portion of the recoater (126) being moved in the scanning direction, energize the drive line (320) to generate a plurality of response signals (416) based on eddy currents induced into one or more consolidated layers (137) of a powder material (118) by the drive line (320); and
process the plurality of response signals (416) to obtain a representation of at least one characteristic of the one or more consolidated layers (137).

3. The system (100) of claim 2, wherein the controller (104) is configured to process the plurality of response signals (416) by passing each of the plurality of response signals (416) through at least one of a high pass median filter (424) or a low pass median filter (426).

4. The system (100) of claim 2, wherein the controller (104) is configured to energize the drive line (320) at one or more frequencies.

5. The system of claim 2, wherein the controller (104) is configured to:
energize the drive line (320) at one or more frequencies; and
for each frequency of the one or more frequencies, obtain data from at least the first row sensor element group (210) and combine the obtained data.

6. The system (100) of claim 2, wherein the controller (104) is configured to evaluate a phase angle of the plurality of response signals (416) to obtain the representation of the at least one characteristic of the one or more consolidated layers (137).

7. The system (100) of claim 1, wherein:
the first row sensor element group (210) comprises one or more first sensor elements (302) arranged along a first linear axis (220);
the second row sensor element group (212) comprises on or more second sensor elements (306) arranged along a second linear axis (222); and
the third row sensor element group (214) comprises one or more third sensor elements (310) arranged along a third linear axis (224), wherein the first, second, and third linear axes (220, 222, 224) are orthogonal to a scanning direction.

8. The system (100) of claim 7, wherein the first, second, and third linear axes (220, 222, 224) are offset from each other in the scanning direction.

9. A method of monitoring an additive manufactured object (114), the method comprising:
moving a recoater (126) in a scanning direction over at least a portion of one or more consolidated layers (137) of a powder material (118) on a build platform (130), wherein the recoater (126) comprises an eddy current array probe (ECAP) (154);
during at least a portion of the moving of the recoater (126), energizing the ECAP (154) to generate a plurality of response signals (416) based on eddy currents induced into the one or more consolidated layers (137) by the ECAP (154);
determining a magnitude of each of the plurality of response signals (416);
determining an effective amplitude of a potential anomaly in the one or more consolidated layers (137) based on the determined magnitude of each of the plurality of response signals (416); and
verifying that the effective amplitude exceeds a threshold.

10. The method of claim 9, further comprising evaluating a phase angle of the plurality of response signals (416) to determine the potential anomaly.

11. The method of claim 9, further comprising passing each of the plurality of response signals (416) through at least one of a high pass filter (424) or a low pass filter (426).

12. The method of claim 9, wherein the ECAP (154) comprises a plurality of sensor element group rows (210, 212, 214), and wherein the method further comprises determining whether the potential anomaly is detected in at least two of the plurality of sensor element group rows (210, 212, 214).

13. The method of claim 9, further comprising:
energizing the ECAP (154) at one or more frequencies; and
determining, for each of the one or more frequencies, the magnitude of each of the plurality of response signals (416).

14. The method of claim 9, wherein the ECAP (154) comprises a plurality of sensor element group rows (210, 212, 214), and wherein the method further comprises:
creating a scan image for each of the plurality of sensor element group rows (210, 212, 214); and
combining the scan image from each of the plurality of sensor element group rows (210, 212, 214) to form a final scan image.

15. The method of claim 9, wherein the ECAP (154) comprises at least one sensor element group row (210, 212, 214), wherein the at least one sensor element group row (210, 212, 214) includes a plurality of sensor elements (302, 306, 310), and wherein the method further comprises determining whether the potential anomaly is detected by at least two adjacently disposed sensor elements (302, 306, 310) of the plurality of sensor elements (302, 306, 310).
